# EUROPÄISCHE PATENTSCHRIFT

(11) **EP 3 881 052 B1**
(45) Veröffentlichungstag und Bekanntmachung des Hinweises auf die Patenterteilung: **26.06.2024**
(21) Anmeldenummer: 19769780.8
(22) Anmeldetag: 16.09.2019
(51) Int. Cl.: G01N 15/06, G01N 33/00

(54) **VERFAHREN ZUM BEWERTEN DER FUNKTIONSFÄHIGKEIT EINES SENSORS ZUR DETEKTION VON RUSS**
METHOD FOR ASSESSING THE OPERATABILITY OF A SENSOR FOR DETECTING SOOT
PROCÉDÉ D'ÉVALUATION DE L'APTITUDE AU FONCTIONNEMENT D'UN CAPTEUR DE DÉTECTION DE LA SUIE

(30) Priorität: 16.11.2018 DE 102018219625
(43) Veröffentlichungstag der Anmeldung: 22.09.2021
(73) Patentinhaber: Robert Bosch GmbH, 70442 Stuttgart (DE)
(72) Erfinder: SCHILLING, Carolin Maria, 76669 Bad Schoenborn (DE); KLENK, Mathias, 74369 Loechgau (DE); HERWEG, Karola, 70469 Stuttgart (DE)
(86) Internationale Anmeldenummer: PCT/EP2019/074619
(87) Internationale Veröffentlichungsnummer: WO 2020/098995

(56) Entgegenhaltungen:
- DE-A1-102012 210 525
- DE-A1-102014 220 398
- US-A1- 2011 314 899

## Beschreibung

### Stand der Technik

Aus der DE 10 2012 210 525 A1 der Anmelderin ist bereits ein Verfahren und eine Vorrichtung zur Detektion von Rußpartikeln im Abgas von Brennkraftmaschinen bekannt. Weitere Methoden aus dem Stand der Technik sind in folgenden Dokumenten offengelegt: US 2011/314899A1 und DE102014220398A1.

Es sind insbesondere keramische resistive Ruß-Sensoren mit zwei einem Abgas aussetzbaren Messelektroden und einem Widerstands-Heizelement bekannt. Die Messung einer Rußkonzentration im Abgas basiert hierbei auf der Anlagerung der Rußpartikel zwischen den Messelektroden und einem infolgedessen verringerten elektrischen Widerstand zwischen den Messelektroden. Mit dem Widerstands-Heizelement lassen sich die Rußpartikel abbrennen, sodass eine neue Messung begonnen werden kann.

Es ist ferner bekannt, die Integrität der Elektroden und somit die Funktionalität des Sensors zu überwachen, indem nach einer Regeneration, wenn also eine leitende Verbindung durch Ruß ausgeschlossen ist, die Temperatur des Sensors mit einem Widerstands-Heizelement konstant gehalten wird und währenddessen an die Elektroden eine Spannung angelegt wird, und die Elektroden als intakt bewertet werden, wenn ein resultierender Messstrom oberhalb eines Schwellwertes liegt. Auch ein Messverfahren, dass zwei Messungen der resultierenden Messströme bei zwei verschiedenen Temperaturen des Sensors vorsieht, ist in der DE 10 2012 210 525 A1 offenbart.

### Offenbarung der Erfindung

Die Erfindung basiert auf der Erkenntnis der Erfinder, dass die Messung eines Stroms zwischen den Messelektroden des keramischen Sensors zum Zweck der Bewerten der Funktionsfähigkeit eines Sensors potenziell durch eine Beheizung des Sensors während der Messung verfälscht sein kann. Es wurde insbesondere erkannt, dass die an dem elektrischen Widerstands-Heizelement angelegten Spannungen bzw. Potenziale Ionen in dem Sensor zu verschieben vermögen, sodass hieraus elektrische Ströme zu den Messelektroden bzw. zwischen den Messelektroden bzw. zu einem mit den Messelektroden verbundenen Steuergerät resultieren, die die eigentlich beabsichtigte Messung des mit der zwischen den Messelektroden angelegten Messspannung korrelierten Messstroms verfälschen. Es wurde insbesondere auch erkannt, dass Ein- und Ausschaltvorgänge des Widerstands-Heizelements, wie sie beispielsweise mit der Einregelung der Sensortemperatur auf eine konstante Temperatur einhergehen, zu Störungen des Messstroms führen.

Erfindungsgemäß ist daher vorgesehen, nach dem Freibrennen der Messelektroden von Ruß das Widerstands-Heizelement zu deaktivieren, sodass die elektrische Heizleistung während den nachfolgenden Schritten des erfindungsgemäßen Verfahrend OW beträgt und es keine Bewegung von Ionen in dem Sensor mehr verursacht. Nach einem Abwarten kann dann bei deaktiviertem Widerstands-Heizelement eine Messung einer ersten den elektrischen Widerstand zwischen den Messelektroden repräsentierenden Größe mit hoher Genauigkeit durchgeführt werden. Infolgedessen kann auch die Bewertung der Funktionsfähigkeit des Sensors mit erhöhter Sicherheit durchgeführt werden.

Im Rahmen der Anmeldung werden unter elektrischen Widerständen Größen betrachtet, die sich durch Division einer Spannung mit einem Strom ergeben, also beispielsweise die Einheit Ohm haben.

Im Rahmen der Anmeldung werden unter Größen, die einen elektrischen Widerstand repräsentieren, auch Größen verstanden, die zumindest im vorliegenden Kontext mit einem elektrischen Widerstand eineindeutig zusammenhängen. Beispielsweise kann es sich bei der den elektrischen Widerstand repräsentierenden Größe um den elektrischen Widerstand handeln oder es kann sich bei der den elektrischen Widerstand repräsentierenden Größe im Kontext gegebener Spannung um einen Strom oder um einen inversen Strom handeln. In einem anderen Beispiel kann es sich im Kontext eines gegebenen Stroms bei der den elektrischen Widerstand repräsentierenden Größe um eine Spannung handeln.

Bei dem Abwarten kann es sich um eine vorgegebene Wartezeit handeln, beispielsweise eine Wartezeit von mindestens 50ms und/oder um eine Wartezeit von höchstens einer Sekunde.

Bei dem Abwarten kann es sich aber auch darum handeln, dass abgewartet wird, bis sich der Sensor auf eine vorgegebene Temperatur abgekühlt hat und ein von dem Sensor empfangenes, die Sensortemperatur repräsentierendes Signal einen entsprechende ersten vorgegebenen Wert erreicht. Diese Temperatur kann beispielsweise 780°C oder einen Wert zwischen 700°C und 800°C betragen.

Das die Sensortemperatur repräsentierende Signal kann beispielsweise ein einen elektrischen Widerstand repräsentierendes Signal sein, siehe oben. Der Sensor kann beispielsweise ein Temperaturmesselement, zum Beispiel ein Pt100 oder dergleichen, aufweisen und durch das erfindungsgemäße Verfahren entsprechend angesteuert werden.

Gemäß einer Weiterbildung der Erfindung ist vorgesehen, dass nach der Messung der ersten den elektrischen Widerstand zwischen den Messelektroden repräsentierenden Größe ein zweites Abwarten erfolgt und dann die Messung einer zweiten den elektrischen Widerstand zwischen den Messelektroden repräsentierenden Größe erfolgt und nachfolgend die Bewertung der Funktionsfähigkeit des Sensors auf Basis der ersten den elektrischen Widerstand zwischen den Messelektroden repräsentierenden Größe und auf Basis der zweiten den elektrischen Widerstand zwischen den Messelektroden repräsentierenden Größe vorgenommen wird. Auf diese Weise können Offseteffekte eliminiert werden und die Sicherheit der Bewertung der Funktionsfähigkeit des Sensors weiter verbessert werden. Beispielsweise kann die zweite den elektrischen Widerstand zwischen den Messelektroden repräsentierende Größe von der ersten den elektrischen Widerstand zwischen den Messelektroden repräsentierenden Größe subtrahiert werden und die Bewertung der Funktionsfähigkeit des Sensors auf Basis der resultierenden Differenz erfolgen.

Dass sich die Temperatur des Sensors nach dem Deaktivieren des Widerstands-Heizelement im Allgemeinen vermindert, kann dadurch kompensiert werden, dass der Sensor zuvor auf eine Übertemperatur aufgeheizt wird. Beispielsweise kann das Aufheizen des Sensors zum Freibrennen der zwei Messelektroden von Ruß auf eine erste vorgegebene Temperatur erfolgen, die für mindestens 5s gehalten wird, und die im Bereich zwischen 700°C und 850°C liegt, und vor dem Deaktivieren des Widerstands-Heizelement eine weitere Aufheizung des Sensors mit dem Widerstands-Heizelement auf eine vorgegebene Übertemperatur erfolgen, die mindestens 10K höher ist als die erste vorgegebene Temperatur, also beispielsweise zwischen 710°C und 860°C liegt. Es kann vorgesehen sein, dass die Übertemperatur nicht höher als 40K über der ersten vorgegebenen Temperatur liegt.

Die Erfinder haben überdies erkannt, dass es bei der Messung der ersten den elektrischen Widerstand zwischen den Messelektroden repräsentierenden Größe zu transienten Effekten kommen kann, die wiederum zu Messungenauigkeiten führen können. Beispielsweise wurde beobachtet, dass unmittelbar nach Anlegen oder Vergrößern der Messspannung zwischen den Messelektroden kurzzeitig besonders hohe Ströme aus der Bewegung von Ionen im Sensor resultieren.

Die Erfindung vermeidet diese Effekte und erhöht somit die Sicherheit der Bewertung der Funktionsfähigkeit des Sensors. Sie sieht vor, dass bereits während dem Aktivieren des Widerstands-Heizelement zum Aufheizen des Sensors und zum Freibrennen der zwei Messelektroden von Ruß zwischen den Messelektroden eine Spannung angelegt wird. Diese Spannung wird dann während der nachfolgenden Verfahrensschritte konstant und ununterbrochen beibehalten, also insbesondere während dem Deaktivieren des Widerstands-Heizelements, während dem Abwarten bis ein von dem Sensor empfangenes, die Sensortemperatur repräsentierendes Signal einen ersten vorgegebenen Wert erreicht, während der Messung einer ersten den elektrischen Widerstand zwischen den Messelektroden repräsentierenden Größe und gegebenenfalls während weitere Verfahrensschritte, die dem Bewerten der Funktionsfähigkeit des Sensors vorangehen.

Es hat sich in Untersuchungen der Anmelderin als günstig herausgestellt, wenn diese angelegte Spannung relativ hoch ist, also beispielsweise mehr als 12V beträgt. Sie kann beispielsweise auch mehr als 40V betragen.

Die Erfindung umfasst auch ein Computerprogramm, welches dazu eingerichtet ist, die Schritte des erfindungsgemäßen Verfahrens durchzuführen, einen nichtflüchtigen Speicher, auf welchem ein derartiges Computerprogramm gespeichert ist und eine elektronische Steuerungseinheit, welche einen solchen nichtflüchtigen Speicher umfasst.

Es zeigen:
- Fig. 1: einen keramischen Sensor zur Detektion von Ruß,
- Fig. 2a und 2b: Zeitdiagramme des erfindungsgemäßen Verfahrens.

### Ausführungsformen der Erfindung

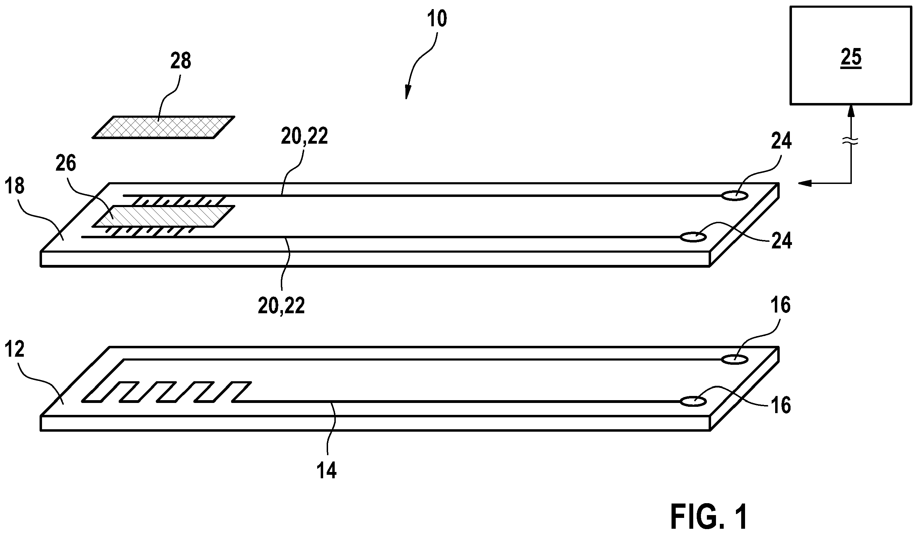

Fig. 1 zeigt einen Sensor 10 zur Detektion von Ruß, in einem Gasstrom, wie beispielsweise einem Abgasstrom einer Brennkraftmaschine, der zum Einbau in einen Abgasstrang eines Kraftfahrzeugs dient. Beispielsweise ist der Sensor 10 stromabwärts eines Rußfilters eines Kraftfahrzeugs mit einem Dieselverbrennungsmotor angeordnet.

Der Sensor 10 umfasst eine plattenförmige Trägerschicht 12, die zumindest teilweise aus einem elektrisch isolierenden Werkstoff hergestellt ist, wie beispielsweise aus einer Keramik wie Aluminiumoxid. In die Trägerschicht 12 ist ein Widerstands-Heizelement 14 integriert, das über Kontaktierungen 16 mit einer geeigneten Spannungsquelle verbindbar ist und zum Freibrennen des Sensors 10 von gegebenenfalls abgelagerten Teilchen, wie Rußpartikeln, dient.

Auf der Trägerschicht 12 ist ein plattenförmiges Substrat 18 angeordnet, das zumindest teilweise aus einem elektrisch isolierenden Werkstoff hergestellt ist, wie beispielsweise einer Keramik wie Aluminiumoxid. Auf dem Substrat 18 ist eine Struktur aus zwei Messelektroden 20 angeordnet. Beispielsweise sind die Messelektroden 20 als Interdigitalelektroden 22 ausgebildet, so dass sie kammförmig ineinandergreifen. Die Messelektroden 20 sind über Kontaktierungen 24 mit einer Steuerung 25 verbindbar.

In dem Bereich, in dem die Messelektroden 20 kammförmig ineinandergreifen, können die Messelektroden 20 zumindest teilweise von einem Dielektrikum 26 überdeckt sein, so dass die Messelektroden 20 als Elektroden eines Kondensators mit messbarer Kapazität dienen können. Das Dielektrikum 26 kann wiederum mit einer Schutzschicht 28 versehen sein, so dass es gegenüber dem umgebenden Medium abgetrennt ist, wodurch eine Degeneration des Dielektrikums 26 ausgeschlossen ist.

Der Sensor 10 kann ferner ein Gehäuse umfassen, das den in Fig. 1 dargestellten Aufbau umgibt und in Fig. 1 nicht gezeigt ist.

Der Sensor 10 nach der Fig. 1 kann wie folgt arbeiten. Wenn sich auf dem Substrat 18 Ruß bzw. sonstige elektrisch leitende Teilchen ablagern, so reduziert sich ein elektrischer Widerstand zwischen den beiden Messelektroden 20. Durch Messung der Impedanz zwischen den beiden Messelektroden 20 ergibt sich ein für ein RC-Glied typisches Verhalten. Dies bedeutet, dass die Ruß- bzw. Teilchenkonzentration in dem betreffenden Abgas anhand der zeitlichen Änderung des Widerstandsanteils des RC-Gliedes bestimmt werden kann.

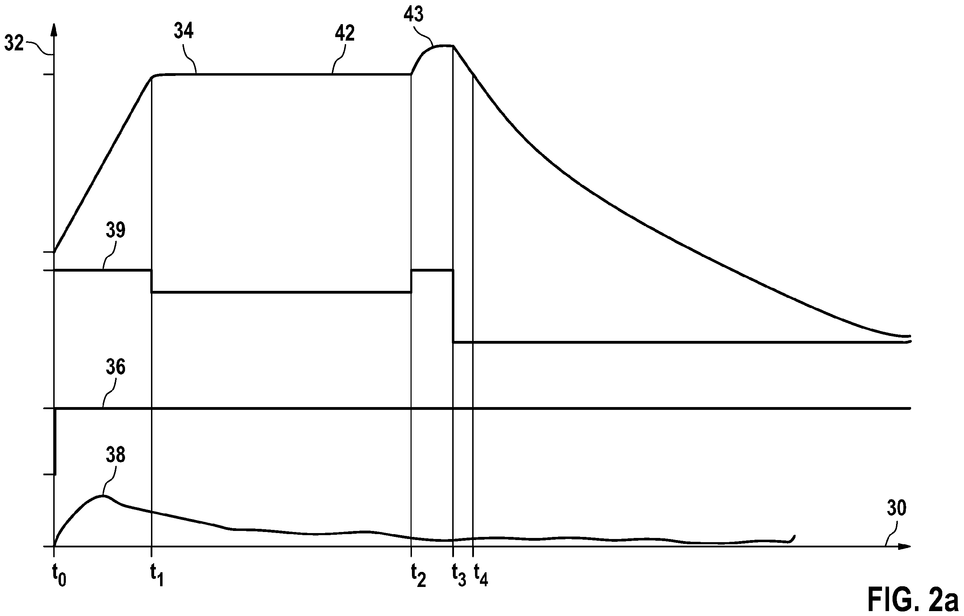

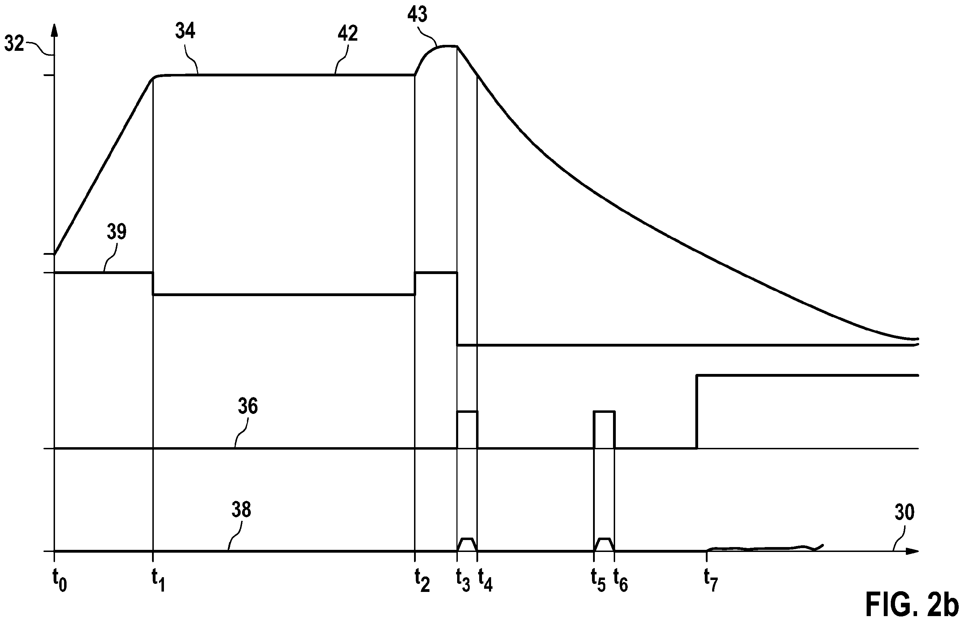

Zur Regeneration des Sensors 10 werden die angelagerten Teilchen nach gewisser Zeit mittels des in die Trägerschicht 12 integrierten Widerstands-Heizelements 14 abgebrannt. Bei funktionstüchtigem Sensor 10 sollte nach diesem Ausheizen der Widerstand zwischen den Messelektroden 20 deutlich ansteigen und durch die Leitfähigkeit der die Messelektroden 20 umgebenden Schichten des Sensors 10 gegeben sein. Da die Arbeitsweise des Sensors 10 zur Detektion der Teilchenkonzentration an sich bekannt ist, beispielsweise aus der WO 2003/006976 A2, wird an dieser Stelle nicht näher auf die gewöhnliche Arbeitsweise des Sensors 10 eingegangen und der Inhalt des oben genannten Stands der Technik, der die Beschreibung der Funktionsweise des Sensors 10 betrifft, ist durch Verweis hierin vollständig eingeschlossen. Stattdessen wird nun das erfindungsgemäße Verfahren zur Funktionskontrolle des Sensors 10 beschrieben. Das Verfahren kann beispielsweise von der oben genannten Steuerung 25 durchgeführt werden. Insbesondere wird das Verfahren anhand der Figuren 2a und 2b beschrieben.

Insbesondere ist in Fig. 2a die Zeit auf der X-Achse 30 dargestellt und auf der Y-Achse 32 der Verlauf bestimmter Größen, wie einer Temperatur 34, einer zwischen den Messelektroden 20 anliegenden elektrischen Spannung 36 und eines zwischen den Messelektroden 20 fließenden elektrischen Stroms 38 sowie einer Heizleistung 39 des Widerstands-Heizelements 14 über der Zeit dargestellt.

Fig. 2a zeigt beispielsweise das oben beschriebene Freibrennen oder Ausheizen des Sensors 10. Dazu wird der Sensor 10 mittels des Widerstands-Heizelements 14 erhitzt, wie beispielsweise anhand des Verlaufs der Temperatur 34 ab dem ersten Zeitpunkt t0 erkennbar ist. Der Sensor 10 wird erhitzt bis zum Erreichen einer ersten vorgegebenen Temperatur 42 zum Zeitpunkt. Die erste Temperatur 42 wird ab dem Zeitpunkt t1 bis zum Zeitpunkt t2 beispielsweise über einen Zeitraum von 45 s konstant gehalten und beträgt beispielsweise 785 °C. Da die Temperatur zwischen den Zeitpunkten t1 und t2 nicht weiter erhöht wird, ist die Heizleistung 39 während dieser Zeit geringer als zwischen den Zeitpunkten t0 und t1.

Zwischen den Zeitpunkten t2 und t3 wird die Heizleistung wieder erhöht, beispielsweise auf den gleichen Wert wie zwischen den Zeitpunkten t0 und t1. Infolgedessen steigt die Temperatur auf eine Übertemperatur 43, beispielsweise auf 805°C zum Zeitpunkt t3.

Zum Zeitpunkt t3 wird das elektrische Widerstands-Heizelement 14 deaktiviert, das heißt, die elektrische Heizleistung 39 beträgt nachfolgend und innerhalb des verbleibenden Messzyklus bzw. während der weiteren Schritte des Verfahrens zum Bewerten der Funktionsfähigkeit des Sensors stets den Wert 0W. Aufgrund der thermischen Kopplung des Sensors 10 an seine Umgebung kühlt er sich ab. Beispielsweise erreicht er 650 ms später, zum Zeitpunkt t4, wieder die Temperatur 785°C.

Es ist vorgesehen, dass nun, zum Zeitpunkt t4, die Messung einer ersten den elektrischen Widerstand zwischen den Messelektroden 20 repräsentierenden Größe erfolgt, beispielsweise die Messung des zwischen den Messelektroden 20 fließenden elektrischen Stroms 38.

Ferner ist in Fig. 2a der Verlauf der zwischen den Messelektroden 20 angelegten elektrischen Spannung 36 dargestellt. In diesem Beispiel beträgt diese Spannung ab dem Zeitpunkt t0 bis nach dem Zeitpunkt t4 konstant 45,6 V.

Aus dem gemessenen zwischen den Messelektroden 20 fließenden elektrischen Strom 38 und der bekannten zwischen den Messelektroden 20 angelegten elektrischen Spannung 36 kann auf den elektrischen Widerstand zwischen den Messelektroden 20 geschlossen werden.

Ist dieser Widerstand kleiner als ein vorgegebener Schwellwert, wird der Sensor als funktionsfähig bewertet. Ist dieser Widerstand hingegen größer als der vorgegebene Schwellwert, wird der Sensor als nicht funktionsfähig bewertet. Es kann beispielsweise ein entsprechender Eintrag in einem Fehlerspeicherregister vorgenommen werden oder eine Motorkontrollleuchte (MIL; Malfunction Indicator Light) angesteuert werden, um einen Fehler anzuzeigen.

Nach dem Zeitpunkt t4 kühlt sich der Sensor 10 weiter ab und unterhalb einer bestimmten Temperatur kann sich wieder Ruß an den Messelektroden 20 ablagern. Hierdurch beginnt bereits der nächste Messzyklus.

Ein abgewandeltes Ausführbeispiel ist in der Figur 2b dargestellt. Es unterscheidet sich von dem zuvor erläuterten Ausführbeispiel zunächst darin, dass die zwischen den Messeelektroden 20 anliegende Messspannung 36 zwischen den Zeitpunkten t0 und t3 den Wert 0V hat und zwischen den Zeitpunkten t3 und t4 den Wert 7,5V hat. Dieser Wert wird zur Bestimmung des ersten elektrischen Widerstands verwendet. Zum Zeitpunkt t7, zu dem der nächste Messzyklus beginnt, wird die die zwischen den Messeelektroden 20 anliegende Messspannung 36 auf den Wert 45,6V geschaltet.

Das in der Figur 2b dargestellte Ausführbeispiel unterscheidet sich ferner dadurch von dem eingangs erläuterten Beispiel, dass zu einem Zeitpunkt t5, eine feste Zeit nach dem Zeitpunkt t4 oder bei Erreichen des Sensors 10 einer zweiten Temperatur eine zweite den elektrischen Widerstand zwischen den Messelektroden 20 repräsentierenden Größe bestimmt wird. Dazu wird ähnlich wie zwischen den Zeitpunkten t3 und t4 die zwischen den Messeelektroden 20 anliegende Messspannung 36 bis zum Zeitpunkt t6 auf den Wert 7,5V geschaltet. Die Bewertung der Funktionsfähigkeit des Sensors 10 erfolgt auf Basis des ersten Widerstands und des zweiten Widerstands, beispielsweise auf Basis der Differenz dieser Größen.

## Patentansprüche

1. Verfahren zum Bewerten der Funktionsfähigkeit eines keramischen Sensors (10) zur Detektion von Ruß, wobei der Sensor (10) zwei einem Abgas aussetzbare, voneinander beabstandete Messelektroden (20) und ein elektrisches Widerstands-Heizelement (14) umfasst, wobei das Verfahren die folgenden Schritte umfasst:
• Aktivieren des Widerstands-Heizelements (14) zum Aufheizen des Sensors (10) und zum Freibrennen der zwei Messelektroden (20) von Ruß, nachfolgend
• Deaktivieren des Widerstands-Heizelements (14), nachfolgend
• Abwarten einer ersten vorgegebenen Zeitdauer und/oder Abwarten bis ein von dem Sensor (10) empfangenes, die Sensortemperatur (34) repräsentierendes Signal einen ersten vorgegebenen Wert erreicht, nachfolgend
• Messung einer ersten den elektrischen Widerstand zwischen den Messelektroden (20) repräsentierenden Größe, nachfolgend
• Bewerten der Funktionsfähigkeit des Sensors (10) auf Basis der ersten den elektrischen Widerstand zwischen den Messelektroden (20) repräsentierenden Größe, **dadurch gekennzeichnet dass**, während dem Aktivieren des Widerstands-Heizelements (14) zum Aufheizen des Sensors (10) und zum Freibrennen der zwei Messelektroden (20) von Ruß zwischen den Messelektroden (20) eine Spannung (36) angelegt und in den nachfolgenden Verfahrensschritten beibehalten wird.

2. Verfahren nach Anspruch 1, **dadurch gekennzeichnet, dass** nach der Messung der ersten den elektrischen Widerstand zwischen den Messelektroden (20) repräsentierenden Größe folgendes erfolgt:
• Abwarten einer zweiten vorgegebenen Zeitdauer oder Abwarten bis ein von dem Sensor (10) empfangenes, die Sensortemperatur (34) repräsentierendes Signal einen zweiten vorgegebenen Wert erreicht, nachfolgend
• Messung einer zweiten den elektrischen Widerstand zwischen den Messelektroden (20) repräsentierenden Größe, nachfolgend
• Bewerten der Funktionsfähigkeit des Sensors (10) auf Basis der ersten den elektrischen Widerstand zwischen den Messelektroden (20) repräsentierenden Größe und auf Basis der zweiten den elektrischen Widerstand zwischen den Messelektroden (20) repräsentierenden Größe.

3. Verfahren nach einem der vorhergehenden Ansprüche, **dadurch gekennzeichnet, dass** das Aufheizen des Sensors (10) zum Freibrennen der zwei Messelektroden (20) von Ruß auf eine erste vorgegebene Temperatur erfolgt, die für mindestens 5s gehalten wird, und im Bereich zwischen 700°C und 850°C liegt.

4. Verfahren nach Anspruch 3, **dadurch gekennzeichnet, dass** vor dem Deaktivieren des Widerstands-Heizelements (14) eine weitere Aufheizung des Sensors (10) mit dem Widerstands-Heizelement (14) auf eine vorgegebene Übertemperatur (43) erfolgt, die mindestens 10K höher ist als die erste vorgegebene Temperatur.

5. Verfahren nach einem der vorhergehenden Ansprüche, **dadurch gekennzeichnet, dass** die während dem Aktivieren des Widerstands-Heizelements (14) zum Aufheizen des Sensors (10) und zum Freibrennen der zwei Messelektroden (20) von Ruß zwischen den Messelektroden (20) angelegte Spannung (36) mehr als 12V beträgt.

6. Computerprogramm, welches dazu eingerichtet ist, in Kombination mit einem keramischen Sensor zur Detektion von Ruß, wobei der Sensor (10) zwei einem Abgas aussetzbare, voneinander beabstandete Messelektroden (20) und ein elektrisches Widerstands-Heizelement (14) umfasst, um die Schritte des Verfahrens nach einem der vorangehenden Ansprüche durchzuführen.

7. Nichtflüchtiger Speicher, auf welchem ein Computerprogramm nach dem vorangehenden Anspruch gespeichert ist.

8. Elektronische Steuerungseinheit (25), welche einen nichtflüchtigen Speicher nach dem vorangehenden Anspruch umfasst.

## Claims

1. Method for assessing the operability of a ceramic sensor (10) for detecting soot, wherein the sensor (10) comprises two measurement electrodes (20), which are spaced apart from one another and can be exposed to an exhaust gas, and an electrical resistance heating element (14), wherein the method comprises the following steps:
• activating the resistance heating element (14) for heating the sensor (10) and for burning soot off the two measurement electrodes (20), subsequently
• deactivating the resistance heating element (14), subsequently
• waiting for a first predefined period of time and/or waiting until a signal, which is received from the sensor (10) and represents the sensor temperature (34), reaches a first predefined value, subsequently
• measuring a first variable representing the electrical resistance between the measurement electrodes (20), subsequently
• assessing the operability of the sensor (10) on the basis of the first variable representing the electrical resistance between the measurement electrodes (20),
**characterized in that**
a voltage (36) is applied between the measurement electrodes (20) during the activation of the resistance heating element (14) for heating the sensor (10) and for burning soot off the two measurement electrodes (20) and said voltage is maintained in the subsequent method steps.

2. Method according to Claim 1, **characterized in that**, after the first variable representing the electrical resistance between the measurement electrodes (20) has been measured, the following steps are carried out:
• waiting for a second predefined period of time or waiting until a signal, which is received from the sensor (10) and represents the sensor temperature (34), reaches a second predefined value, subsequently
• measuring a second variable representing the electrical resistance between the measurement electrodes (20), subsequently
• assessing the operability of the sensor (10) on the basis of the first variable representing the electrical resistance between the measurement electrodes (20) and on the basis of the second variable representing the electrical resistance between the measurement electrodes (20) .

3. Method according to either of the preceding claims, **characterized in that** the sensor (10) is heated to burn soot off the two measurement electrodes (20) at a first predefined temperature which is retained for at least 5 s and is in the range between 700°C and 850°C.

4. Method according to Claim 3, **characterized in that**, before the resistance heating element (14) is deactivated, the sensor (10) is heated further using the resistance heating element (14) to a predefined excess temperature (43) which is at least 10 K higher than the first predefined temperature.

5. Method according to one of the preceding claims, **characterized in that** the voltage (36) applied between the measurement electrodes (20) during the activation of the resistance heating element (14) for heating the sensor (10) and for burning soot off the two measurement electrodes (20) is more than 12 V.

6. Computer program, which is configured to carry out the steps of the method according to one of the preceding claims in combination with a ceramic sensor for detecting soot, wherein the sensor (10) comprises two measurement electrodes (20), which are spaced apart from one another and can be exposed to an exhaust gas, and an electrical resistance heating element (14).

7. Non-volatile memory, on which a computer program according to the preceding claim is stored.

8. Electronic control unit (25), which comprises a non-volatile memory according to the preceding claim.

## Revendications

1. Procédé d'évaluation de l'aptitude au fonctionnement d'un capteur en céramique (10) destiné à la détection de la suie, le capteur (10) comprenant deux électrodes de mesure (20) espacées l'une de l'autre et pouvant être exposées à un gaz d'échappement, et un élément chauffant résistif (14) électrique, le procédé comprenant les étapes suivantes :
• activation de l'élément chauffant résistif (14) pour chauffer le capteur (10) et pour débarrasser par combustion les deux électrodes de mesure (20) de la suie, puis
• désactivation de l'élément chauffant résistif (14), puis
• attente pendant une première durée prédéterminée et/ou attente jusqu'à ce qu'un signal reçu par le capteur (10) et représentant la température du capteur (34) atteigne une première valeur prédéterminée, puis
• mesure d'une première grandeur représentant la résistance électrique entre les électrodes de mesure (20), puis
• évaluation de l'aptitude au fonctionnement du capteur (10) sur la base de la première grandeur représentant la résistance électrique entre les électrodes de mesure (20),
**caractérisé en ce que**,
pendant l'activation de l'élément chauffant résistif (14) pour chauffer le capteur (10) et pour débarrasser par combustion les deux électrodes de mesure (20) de la suie présente entre les électrodes de mesure (20), une tension (36) est appliquée et maintenue pendant les étapes suivantes du procédé.

2. Procédé selon la revendication 1, **caractérisé en ce qu'**après la mesure de la première grandeur représentant la résistance électrique entre les électrodes de mesure (20), il est procédé à ce qui suit :
• attente pendant une seconde durée prédéterminée ou attente jusqu'à ce qu'un signal reçu par le capteur (10) et représentant la température du capteur (34) atteigne une seconde valeur prédéterminée, puis
• mesure d'une seconde grandeur représentant la résistance électrique entre les électrodes de mesure (20), puis
• évaluation de l'aptitude au fonctionnement du capteur (10) sur la base de la première grandeur représentant la résistance électrique entre les électrodes de mesure (20) et sur la base de la seconde grandeur représentant la résistance électrique entre les électrodes de mesure (20).

3. Procédé selon l'une quelconque des revendications précédentes, **caractérisé en ce que** le chauffage du capteur (10) pour débarrasser par combustion les deux électrodes de mesure (20) de la suie est effectué à une première température prédéterminée, maintenue pendant au moins 5s, et comprise entre 700°C et 850°C.

4. Procédé selon la revendication 3, **caractérisé en ce qu'**avant la désactivation de l'élément chauffant résistif (14), un chauffage supplémentaire du capteur (10) est effectué au moyen de l'élément chauffant résistif (14) jusqu'à une température de dépassement prédéterminée (43) qui est supérieure d'au moins 10K à la première température prédéterminée.

5. Procédé selon l'une quelconque des revendications précédentes, **caractérisé en ce que** la tension (36) appliquée pendant l'activation de l'élément chauffant résistif (14) pour chauffer le capteur (10) et pour débarrasser les deux électrodes de mesure (20) de la suie présente entre les électrodes de mesure (20) est supérieure à 12V.

6. Programme d'ordinateur conçu pour être utilisé en combinaison avec un capteur en céramique destiné à la détection de la suie, le capteur (10) comprenant deux électrodes de mesure (20) espacées l'une de l'autre et pouvant être exposées à un gaz d'échappement, et un élément chauffant résistif (14) électrique, pour exécuter les étapes du procédé selon l'une quelconque des revendications précédentes.

7. Mémoire non volatile dans laquelle est stocké un programme d'ordinateur selon la revendication précédente.

8. Unité de commande électronique (25) comprenant une mémoire non volatile selon la revendication précédente.
